# EUROPEAN PATENT APPLICATION

(11) **EP 3 695 739 A1**
(43) Date of publication of application: **19.08.2020**
(21) Application number: 20382114.5
(22) Date of filing: 17.02.2020
(51) Int. Cl.: A41D 27/28, A41D 31/12

(54) **CLOTHING ARTICLE WITH REFRIGERANT INSERT**

(30) Priority: 15.02.2019 ES 201930125
(71) Applicant: Merino Garcia, Eugenio, 28230 Las Rozas (ES); Merino Garcia, José Miguel, 28016 Madrid (ES)
(72) Inventor: Merino Garcia, Eugenio, 28230 Las Rozas (ES); Merino Garcia, José Miguel, 28016 Madrid (ES)
(74) Representative: Elzaburu S.L.P.

(57) **Abstract**

Clothing article mainly for sportswear use, comprising at least one perforated PVA (polyvinyl alcohol) cooling material insert. With this insert the achieved goals are that the clothing that include them have a greater evaporative surface increasing the cooling effect, greater elasticity to allow adaptability to the body and comfort, less weight and better aesthetics.

## Description

### FIELD OF THE INVENTION

The present invention falls within the field of clothing, in particular clothing for sports and/or therapeutic uses, with skin cooling properties so as to help reduce the user's body temperature.

### BACKGROUND

Currently, there are different types of cooling garments, mainly intended for work activities, made in part or in full with absorbent fabrics of polyester fibers or synthetic polymeric materials. Thus, for example, from US 2012/0276332 A1 the use of composite materials in clothing or footwear comprising layers having different properties, such as water repellent, antimicrobial, thermal insulation, and redistribution of body heat, is known.

Other garments are also known that include absorbent or super absorbent materials, such as baby diapers. To this particular, for example, is the document WO 2018/199974 A1, which discloses baby diapers that comprise various layers, some of which may contain absorbent materials.

There are documents in the prior art that have contemplated the perforation of inserts in absorbent articles such as diapers, as described in WO 2018/182601 A1, but in this case the function of the perforations is different, namely to provide channels through which exudate can pass so that they can be absorbed into the absorbent layer adjacent to that of the adsorbent material. This is a function very different from that of the perforated inserts used in the present invention since, in the case of the present invention, the intended function is that, thanks to the adiabatic cooling effect, the user's skin results refrigerated.

Finally, document US 2007/0163027 A1 describes articles of clothing, among which can be found as headbands, wristbands, gloves, shirts, shorts, etc., which include a water absorbent material such as PVA without perforations. This document indicates that these types of articles are intended to reduce body temperature; however, in practice these garments are little or not suitable for sports practice or therapeutic applications, mainly due to the total absence of elasticity of the absorbent material, as well as the poor evaporation of sweat of the mentioned absorbent material, since the sweat absorbed by the absorbent material described in this document must pass through its full thickness before it can reach the outside and evaporate there.

Therefore, it would be desirable to provide articles of clothing, particularly intended for sportswear, that improve the skin's cooling properties known from the prior art, and also provide them with greater elasticity as well as better evaporation.

### SUMMARY OF THE INVENTION

Consequently, the problem to be solved in the present invention is that of providing garments, mainly for sportswear, that improve the cooling properties known from the prior art and that also provide them with greater elasticity.

The solution to this problem is based on the fact that the inventors have identified that it is possible to achieve the indicated objectives by means of articles of clothing that have been provided with inserts made of PVA (polyvinyl alcohol) material, on which a series of perforations have been made. These perforations should be large enough to allow greater elasticity of the clothing article as a whole, but not so much as to weaken the material's resistance to tearing and tensile strength. Likewise, it is also possible that the perforations do not completely cross the entire thickness of the insert, but it may be sufficient for them to partially cross it, if the desired increase in elasticity is already achieved in this way.

In this regard, it should be noted that PVA foam is currently used commercially in products very different from the use given in the present invention, since this material can be found commonly in absorbent strips used in mops for floor cleaning. In this type of products, the liquid absorption capacity of the PVA foam is very advantageous for the intended purpose. However, PVA foam has a peculiarity that when it dries, it becomes a hard, rigid material, rough to the touch and that tends to form wrinkles, which makes it very disadvantageous when used in garments for human use, and more particularly for sportswear, which are usually worn particularly tight to the body, since a rigid and rough material would not only be very unsightly, but fundamentally highly uncomfortable for the athlete.

It is for this reason that, in the prior art documents, the layers that sports garments are sometimes equipped with are not normally made of PVA but of other materials, in order to avoid the disadvantages of PVA foam when it dries.

However, the few documents that, to the knowledge of the inventors, describe layers in sports garments made of PVA foam such as US 2007/0163027 A1 and US2014 / 0223634 A1 do not provide any solution to this problem. In the first of these, this problem is not mentioned, and in the second, although it is recognized that, when the PVA sponge dries, it becomes a relatively hard and rigid material, the only solution that this document contributes to this problem is to soak the PVA sponge layer in water or other liquids, which obviously does not represent any solution to the user when using such a sportswear, since it is not possible to maintain the PVA foam during use permanently moistened without drying in minutes.

In the present invention, this problem has been addressed by a different approach, namely, by perforating the PVA foam in the form of an insert. As the inventors have found, in making such perforations, the garment still largely retains its elasticity even though it is relatively dry, preventing it from becoming a dry, hard and rough material. Obviously, if the PVA foam is moistened, the elasticity and pleasant feel of the material are even better but, in any case, these properties are much higher when the PVA sponge has been perforated than when it is not.

In relation to documents US 2007/0163027 A1 and US2014 / 0223634 A1, although in some cases this problem is recognized, however no solution to it is described. And, on the other hand, in the prior art there are documents in which perforations have been made in different types of clothing components, in particular sports clothing, such as for example documents US2017 / 0332715 A1 or US 2003/0061650 A1. However, in these documents, in which the layers are not made of PVA, the objective of the perforations is to facilitate perspiration from the skin, transferring the user's sweat from the inner layer to the outer fabric, so that it can dissipate. On the contrary, in the present invention, the perforations are intended for a very different purpose, namely, to make the PVA foam, and consequently the sportswear as a whole, have an elasticity that it would not have in the absence of such perforations. Consequently, it would not be obvious to the expert in the previous technique to perforate PVA foams in order to achieve the aforementioned advantages, and in particular the improved elasticity of sportswear containing PVA inserts even when they get dry to some degree.

Therefore, in a first aspect the invention is aimed to a clothing article comprising a main material made of textile, such as cotton or other natural or synthetic materials, and at least one insert attached by at least one of its sides to the mentioned main material, in which the insert consists of a mesh of mainly textile material covered on at least one of its faces by a coating comprising at least 70% by volume of polyvinyl alcohol foam (PVA), characterised in that the insert presents perforations or holes.

In a second aspect, the invention also relates to a container containing the clothing article according to the invention, which allows it to be preserved with a certain relative humidity, necessary for the cooling inserts to retain their elasticity, since as mentioned, when they dry they become stiff.

### DRAWINGS

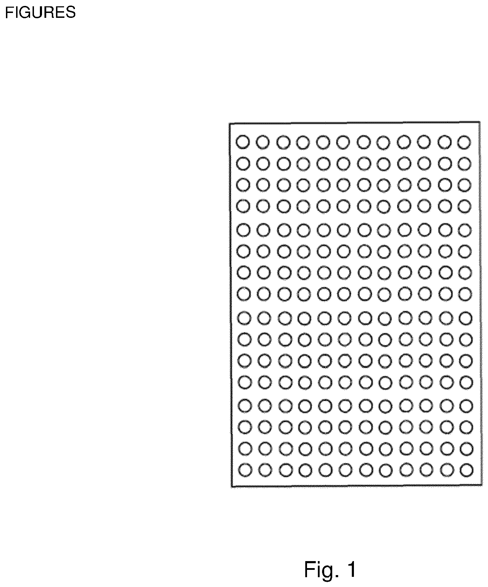

Figure 1 is a schematic image of the cooling inserts described in the present invention, showing the perforations made in them.

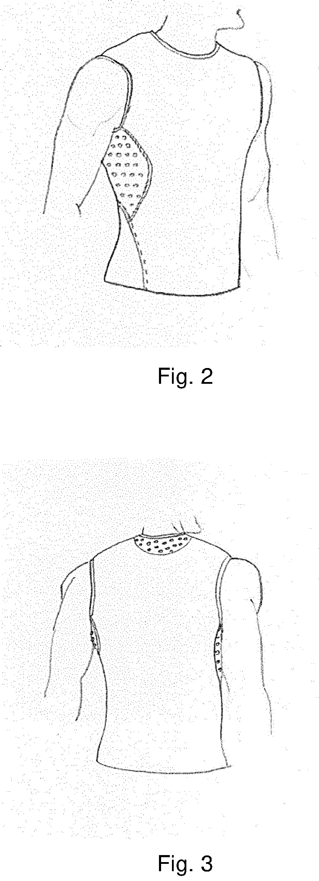

Figure 2 shows a side view of a sportswear garment that includes a perforated absorbent insert in the area of the side under the armpit.
Figure 3 shows a rear view of the sportswear garment of the previous figure, showing perforated absorbent inserts on both sides, as well as in the lower area of the neck.

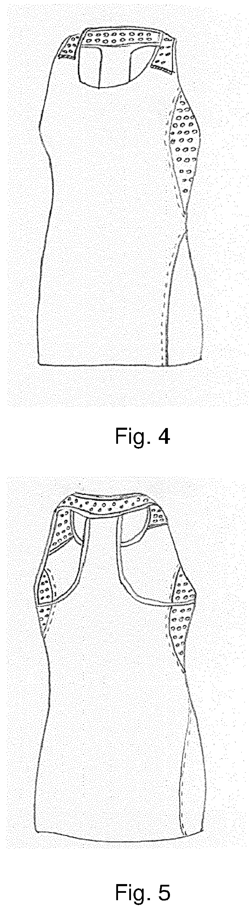

Figures 4 and 5 show a front view of a sportswear garment, which in this case is preferably for female use, and which has perforated absorbent inserts on the sides under the armpit, as well as in the lower area of the neck and shoulders .

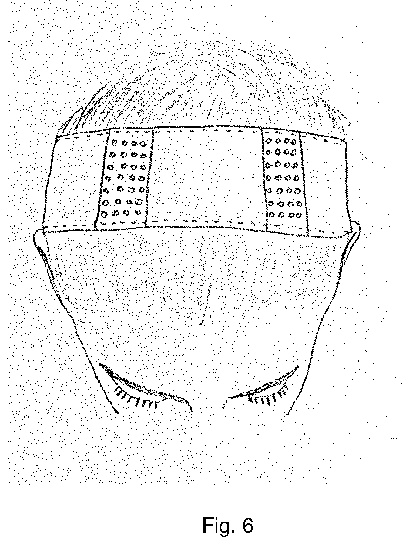

Figure 6 shows a headband containing perforated absorbent inserts in two lateral areas thereof.

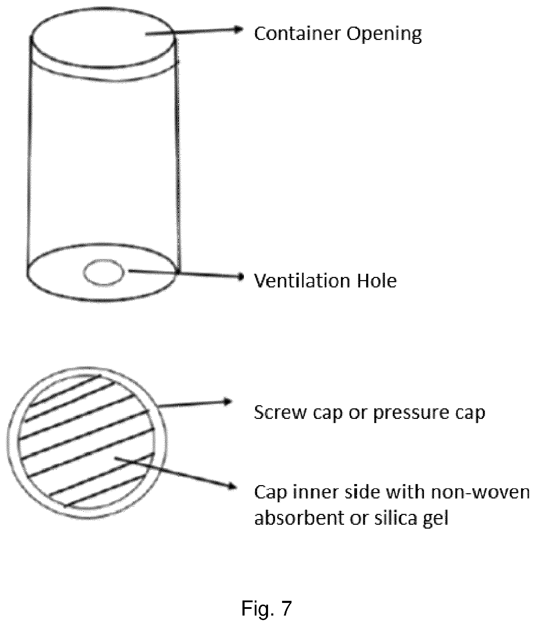

Figure 7 shows a suitable container for keeping the cooling garments according to the invention.

### DEFINITIONS

Prior to the discussion of the detailed description of the invention, the definition of specific terms related to the main aspects of the present invention is provided below.

By "absorbent material" is meant herein a material that is capable of absorbing at least 10 times its weight in water.

"Garment or clothing article" means any clothing article that can be worn by a user. In preferred embodiments, these garments are preferably sportswear garments, including T-shirts, pants (either short or long), headbands, wristbands, training suits, socks, and even underwear or footwear.

By "insert" is meant a piece of material that is inserted in certain areas of articles of clothing and attached to the main material of the clothing article generally by seams that can be of different types.

By "PVA insert" is meant an insert that is made of a mesh of mainly textile material on which a coating made of at least 70% by volume of PVA material in form of foam is applied at least on one of its faces, but preferably both, and which can be composed by up to the remaining 30% by volume by other polymers, among which are polyester and polypropylene, among others. In turn, the mesh of textile material can also carry a proportion of up to 30% by volume of other elastic materials or elastomers such as elastane (also called spandex), materials mentioned by way of illustration only and without limitation thereto. In this way, the resulting mesh has improved elasticity, which can be very advantageous in preferred embodiments of the invention. In the present invention, the mesh assembly plus the coating layer or layers of PVA or other materials are considered to form a single layer or insert, which is why in the present invention this set is called "monolayer". Consequently, when in the present description it is indicated that the insert is "monolayer", it should be understood that it contains a single layer of the indicated material, consisting of a mesh of mainly textile material covered on at least one of its faces by PVA in the form of foam and optionally other materials. It is desirable that the insert used in the present invention is monolayer, that is, it consists of a single layer of material covered on at least one of its faces by a PVA foam coating and optionally other materials. Given that one of the features to be sought by using inserts in the present invention is to provide elasticity to the garment as a whole, if the inserts were made up of several layers of material, in particular layers of inelastic materials or elastic materials which are substantially less elastic than that of the described material, the elasticity of the garment as a whole would be compromised, which would be highly inconvenient for an eminently sports garment. However, it could be feasible that the insert was made up of several layers of PVA coating, since in this way the elasticity would not be compromised.

By "perforation" or "hole" is meant a hole made in one or more pieces of the material that make up the garments described herein and that, in general, can have any shape (circular, square, rectangular, rhomboidal, etc.), although it is preferably circular in shape.

### DETAILED DESCRIPTION OF THE INVENTION

The present invention is based on incorporating one or more inserts of absorbent material into preferably sports and / or therapeutic clothing articles in order to obtain a reduction in the user's thermal load, which causes the user to increase the feeling of comfort, relief and freshness on the one hand, and on the other hand preserves the user's energy, optimizing performance during physical exercise. The inserts used in the garments of the present invention are perforated, which gives them an elasticity that the garments of previous technique do not have, as well as a greater capacity of sweat evaporation.

The garments of the present invention take advantage of the latent heat absorption properties of the water when it evaporates (effect called "adiabatic cooling") of the inserts included therein. The evaporation of water occurs spontaneously by absorbing this heat from the outside, whether in the form of radiation, convection or conduction. Essentially, the inserts produce an adiabatic cooling, as a result of the evaporation of the moisture embedded in the inserts, as they are in contact with the environment and with human skin and, as a final effect, the decrease in body temperature.

Conceptually, the inserts used in the garments of the present invention are water absorbent materials that can contain it molecularly inside, in which a series of perforations have been made, which reduces their weight, increases the evaporative capacity of the absorbed water, and give elasticity to the mentioned material and, therefore, to the clothing article as a whole in which it is inserted.

The absorbent material used in the inserts is polyvinyl alcohol (or PVA), which has undergone a series of perforations, and which is inserted into a garment whose main material is not necessarily moisture absorbent. These main materials can be either natural fabrics such as cotton, or artificial fabrics (so-called "technical fabrics"), in different combinations between them. These fabrics normally have a majority composition of synthetic fibers, preferably polyester, polyamide or any combination of such materials with lower percentages of natural and / or artificial fibers (cotton, cellulosic), and elastomeric fibers to achieve highly elastic fabrics.

The use of absorbent material in the form of elastic inserts integrated into the garment also allows the design of garments that fully adapt to anthropometric measures, notably improving the user's comfort as well as the aesthetics.

The inserts made of absorbent material are intended to be in direct contact with the skin, so they are located in strategic areas of the garment where the cooling effect may be higher, as these areas of the body present a higher density of blood capillaries and nerve endings. Among these areas we can find, without limitation and in an exclusively illustrative way, the following:
- Low area of the nape, under the armpit and under the thorax, ideal for sports shirts;
- Groin area, ideally for sports pants;
- Front area and temples, ideally for sports headbands;
- Circumference or perimeter on sports wristbands;
- External limb rotation zone, ideal for therapeutic knee, ankle and elbow pads.

For the cooling effect to occur, the garment, and especially the cooling inserts, must be in direct contact with the skin, so the most suitable design or trim of the garments is that of the compression or semi-compression type. To improve comfort in this type of garment, flat or "flat-lock" type seams are preferably used, as well as seams glued with elastic adhesive, or even ultrasonic welded seams, and in certain parts of the garment elastic seams are also accepted when attaching laser-cut materials.

In the present invention, as indicated in the "Definitions" section, "PVA inserts" are inserts that are composed of a mesh of mainly textile material on which it is applied, on at least one of its faces, although preferably in both, a coating composed of at least 70% by volume of PVA in the form of a foam, which in turn can be made by up to the remaining 30% by volume by other polymers such as polyester or polypropylene. In turn, the mesh of textile material can also carry a proportion of up to 30% by volume of other elastic or elastomeric materials such as elastane (also called spandex), cited for illustrative purposes only and without limitation thereto. In this way, the resulting mesh has improved elasticity, which can be very advantageous in preferred embodiments of the invention.

In the present invention, the inserts of absorbent material have been perforated punching holes on them, in order to increase the surface in contact with the environment and thus increase the effect of adiabatic cooling, in addition to providing them with elasticity, allowing great adaptability of the garment as a whole, with the added effect of greater comfort and better aesthetics. Another benefit obtained is a notable reduction in the weight of the absorbent material, which by itself, especially when soaked, turns out to be several times heavier than the main fabric of the garment in which it is integrated.

The cooling inserts are perforated with perforations of a predetermined diameter, and ideally spaced to optimize the achievement of four effects: greater evaporative surface to increase the cooling effect, elasticity to allow adaptability to the body and comfort, less weight and better aesthetics.

The perforations or holes in the absorbent material are preferably made using an automatic textile laser on a numerical control machine. The laser sublimates the absorbent material by rapid combustion at high temperature, leaving a very careful finish and without traces of burns, as well as the cauterization of the edges of the perforations, which avoids breaking or fraying at the edges.

Another embodiment of the perforations is by means of a metal plate with spikes, which is mechanically actuated by exerting pressure on the material, although the finishing in this case is inferior to the laser system.

Since the PVA material and as well as the mesh it has inside are substantially inelastic in nature, and since it is desirable to use them in compression or semi-compression garments that require high elasticity, when practicing in the material a series of perforations, it achieves a greater elasticity, while maintaining high values of mechanical resistance so that it cannot be easily broken by traction or tearing.

When using the PVA material as inserts for the manufacturing of cooling garments, the handling of said material must be done keeping it moist, since if it were to dry completely, the PVA would remain rigid and deformed, making its correct application impossible. Therefore, the garment should preferably be stored in containers that maintain a high relative humidity (greater than 85%) inside it, so that the garment is ready for use as soon as it is removed from its container. If not, the item of clothing, and more particularly its inserts, may need to be moistened, for example by dispersing a small amount of water over them, and then wait briefly until the water has been absorbed by the material of the insert.

The inserts used in the present invention are preferably thin, with preferred thicknesses of between 0.5 and 2 mm when dry, and between 1.5 and 3 mm when wet. These inserts contain perforations of different dimensions, which generally must be large and / or numerous enough to carry out their intended function of providing greater flexibility and sweat evaporation, but they must not be so large and / or numerous as to compromise its resistance to tearing. Thus, in general, the spacing of the perforations, measured from the center to the center of two adjacent perforations, may be in the range of 3 to 15 mm, more preferably 4 to 10 mm. It is also possible to vary the diameter of the perforations, which can be between 0.5 and 5 mm, more preferably between 1 and 2 mm. Likewise, within the present invention, different combinations of the parameters relative to the distance between perforations and the diameters of the perforations are considered included, and the possibility that both parameters may be the same, different or even variable within the same insert is contemplated, or even from one insert to another of the same clothing article, with the sole premise that the above conditions regarding elasticity and evaporation on the one hand, and mechanical resistance on the other are met.

In order to establish the optimal relationship and design between perforations, the following significant variables were considered, which were optimized through different tests of tensile strength, garment elasticity, air convection (ventilation) and adiabatic capacity:
- Perforation area
- Distance between holes centers

For the selection of significant versus non-significant variables, the statistical methodology of Analysis of Variance was used.

The optimal number of perforations (as it results from the variables mentioned above) and how these are distributed on the surface of the inserts, was therefore determined experimentally. Based on the tests carried out, it was concluded that optimally, the perforations have a diameter of between 1 and 2 mm, with a typical separation between centers of the perforations between 5 and 10 mm. In this way, based on the fact that the original material has a weight of 415 g / m2, we obtain a significant reduction in weight of 19% with perforations of 2 mm at 8 mm distance, and 27% with perforations of 2 mm at 5 mm distance.

In relation to the container containing the clothing article according to the invention, in order to achieve the relative humidity inside so that the cooling inserts retain their elasticity, in addition to ventilating and preventing the production of bad odors which may develop due to the metabolic activity of fungi and bacteria, the container is preferably provided with a single circular hole, located at its base in those cases in which the container is generally cylindrical in shape, and which has a diameter of between 3 and 6 mm. The physical mechanism sought to promote ventilation is based on the principle of balancing the differential pressure between the inside of the container and the outside environment. Microbial, aerobic and anaerobic activity produces, over time, an increase in the initial pressure of the atmosphere inside the container, due to the molecular transformation of hydrogen, oxygen and nitrogen into organic and inorganic gaseous residues generated by the metabolic activity of bacteria and fungus. Because these gaseous residues are molecules that are at higher different partial pressures, and since the variations in temperature outside the container will also produce different pressures of the atmosphere inside and outside, the hole will act as a shut-off valve balanced fluid dynamics between both atmospheres, allowing the progressive evacuation of part of the interior atmosphere of the container to the outside and, consequently, ventilating the interior.

The container's closure system is preferably a screw cap or a pressure cap, which has on its inner side an absorbent non-woven material or silica salt, helping to balance excess of moisture inside, as well as minimizing germ development. The absorbent material may optionally be impregnated with a perfumed essential oil or a sanitizer for textile use. In this way the garment is ready for use as soon as it is removed from the container.

### EXPERIMENTAL TESTS:

During the period July-August-September 2018, a series of tests were carried out by internet influencers, with temperatures between 30 and 40 ° C and different relative humidity, at different times of the day, with cloudy and sunny conditions, using sportswear garments that included cooling inserts as described above with optimal perforation patterns, i.e. with perforations in diameter between 1 and 2 mm and with a center-to-center separation of perforations between 5 and 10 mm . These runners observed that, in comparison with garments known from the state of the technique, when using garments according to the invention they obtained the following effects:
- Less fatigue.
- Less sweating.
- Greater freshness.
- Improved marks.
- Higher performance.
- Reduced risk of heat stress.

From these tests the benefits obtained by the garments according to the invention are clearly observed.

From the readings of the present description, the skilled person will be able to conceive different variants and combinations between the different explained embodiments. All of these combinations are intended to be included within the scope of the present invention, with no other limitation than that of the attached claims.

## Claims

1. Clothing article comprising a main material made of textile and at least one insert attached by at least one of its sides to the cited main material, wherein the insert consists of a mesh of mostly textile material covered on at least one of its faces by a coating comprising at least 70% by volume of polyvinyl alcohol foam (PVA), **characterised in that** the insert is provided with perforations or holes.

2. Clothing article according to claim 1, wherein the mesh of mostly textile material further comprises other elastic materials such as elastane, also called spandex, in up to 30% by volume of its composition.

3. Clothing article according to claims 1 or 2, wherein the mesh coating contains up to 30% by volume of other polymeric materials such as polyester or polypropylene.

4. Clothing article according to any of claims 1 to 3, wherein the perforations of the insert are separated from each other by a distance of between 3 and 15 mm, measured from center to center thereof.

5. Clothing article according to claim 4, wherein the perforations of the insert are separated from each other by a distance of between 4 and 10 mm, measured from center to center thereof.

6. Clothing article according to any of the preceding claims, wherein the perforations of the insert have a diameter of between 0.5 and 5 mm.

7. Clothing article according to claim 6, wherein the insert perforations have a diameter between 0.5 and 2 mm.

8. Clothing article according to any of the preceding claims, in which all the perforations of the same insert have the same size and spacing between them.

9. Clothing article according to any of the preceding claims 1 to 7, in which the perforations of the same insert have a different size and/or separation between them.

10. Clothing article according to any of the preceding claims, wherein the insert has a thickness of between 0.5 and 2 mm when it is substantially dry.

11. Clothing article according to any of the preceding claims, wherein the insert is attached to the main material of the clothing article in one or more of the following areas of said article:
- Low area of the nape, under the armpit and under the thorax, in the case of sports shirts;
- Groin area in the case of sports pants;
- Front area and temples in the case of sports headbands;
- Circumference or perimeter, in the case of sports wristbands;
- External zone of rotation of the limbs, in the case of knee, ankle and elbow pads.

12. Clothing article according to any of the preceding claims, wherein the inserts are attached to the main material of the clothing article by flat-lock type seams, elastic adhesive bonded seams, ultrasonic welded seams, or elastic bonded seams for attaching laser-cut materials.

13. Clothing article according to any of the preceding claims, which is a clothing article for sports or therapeutic use.

14. Clothing article according to claim 13, selected from the group consisting of T-shirts, shorts or long pants, headbands, wristbands, anklebands, training suits, socks, underwear and footwear.

15. Container comprising at least one clothing article according to any of the preceding claims, which presents at its base or at least one of its walls at least one hole with a diameter between 3 and 6 mm that allows it to keep inside the container a relative humidity of at least 85%.
